# EUROPEAN PATENT SPECIFICATION

(11) **EP 4 261 569 B1**
(45) Date of publication and mention of the grant of the patent: **29.07.2026**
(21) Application number: 23167156.1
(22) Date of filing: 06.04.2023
(51) Int. Cl.: G06N 20/00, G01S 13/88, G01N 33/24, G01S 7/41, A01C 7/20

(54) **DETERMINING SOIL MOISTURE BASED ON RADAR DATA USING A MACHINE-LEARNED MODEL AND ASSOCIATED AGRICULTURAL MACHINES**
BESTIMMUNG DER BODENFEUCHTE BASIEREND AUF RADARDATEN UNTER VERWENDUNG EINES MASCHINENGELERNTEN MODELLS UND ZUGEHÖRIGE LANDWIRTSCHAFTLICHE MASCHINEN
DÉTERMINATION DE L'HUMIDITÉ DU SOL SUR LA BASE DE DONNÉES RADAR À L'AIDE D'UN MODÈLE APPRIS PAR MACHINE ET MACHINES AGRICOLES ASSOCIÉES

(30) Priority: 11.04.2022 US 202217717849
(43) Date of publication of application: 18.10.2023
(73) Proprietor: CNH Industrial Canada, Ltd., Saskatoon SK S7K 3S5 (CA)
(72) Inventor: SCHROEDER, Brittany, Racine, 53404 (US); STANHOPE, Trevor, Oak Lawn, 60453 (US); HENRY, James, Racine, 53404 (US); PREGESBAUER, Michael, A-2514 Traiskirchen (AT)
(74) Representative: CNH Industrial IP Department

(56) References cited:
- LIANG JING ET AL: "Soil Moisture Retrieval Using UWB Echoes via Fuzzy Logic and Machine Learning", IEEE INTERNET OF THINGS JOURNAL, IEEE, USA, vol. 5, no. 5, 14 November 2018 (2018-11-14), pages 3344 - 3352, XP011704923, DOI: 10.1109/JIOT.2017.2760338
- YANG ZHIHUI ET AL: "Soil Moisture Retrieval Using Microwave Remote Sensing Data and a Deep Belief Network in the Naqu Region of the Tibetan Plateau", SUSTAINABILITY, vol. 13, no. 22, 16 November 2021 (2021-11-16), pages 12635, XP093077082, DOI: 10.3390/su132212635
- AROCA RAFAEL V. ET AL: "Calibration of Passive UHF RFID Tags Using Neural Networks to Measure Soil Moisture", JOURNAL OF SENSORS, vol. 2018, 12 April 2018 (2018-04-12), US, pages 1 - 12, XP093077087, ISSN: 1687-725X, DOI: 10.1155/2018/3436503
- ZHANG SHENG ET AL: "Experimental Research on Evaluation of Soil Water Content Using Ground Penetrating Radar and Wavelet Packet-Based Energy Analysis", REMOTE SENSING, vol. 13, no. 24, 12 December 2021 (2021-12-12), pages 5047, XP093077128, DOI: 10.3390/rs13245047

## Description

### FIELD OF THE INVENTION

The present disclosure generally relates to determining soil moisture content and, more particularly, to determining soil moisture content based on radar data using a machine-learned model and associated agricultural machines.

### BACKGROUND OF THE INVENTION

Modern farming practices strive to increase yields of agricultural fields. In this respect, seed-planting implements are towed behind a tractor or other work vehicle to disperse seed throughout a field. For example, seed-planting implements typically include one or more furrow-forming tools or openers that excavate a furrow or trench in the soil. One or more dispensing devices of the seed-planting implements may, in turn, deposit the seeds into the furrow(s). After deposition of the seeds, a furrow-closing assembly may close the furrow in the soil, such as by pushing the excavated soil into the furrow.

The moisture content of the soil within the field is an important parameter when determining the desired depth of the furrow. In this respect, various systems for determining soil moisture content have been developed. While such systems work well, further improvements are needed.

Accordingly, an improved system and method for determining soil moisture content would be welcomed in the technology

YANG ZHIHUI et al: "Soil Moisture Retrieval Using Microwave Remote Sensing Data and a Deep Belief Network in the Naqu Region of the Tibetan plateau", SUSTAINABILITY, vol. 13, no. 22, 16 November 2021, page 12635, XP093077082,DOI: 10.3390/su132212635 discloses the use of a machine leanring model for determining soil moisture.

A wavelet-based approach is known from ZHANG SHENG et al: "Experimental Research on Evaluation of Soil Water Content Using Ground Penetrating Radar and Wavelet Packet-Based Energy Analysis", REMOTE SENSING, vol. 13, no. 24, 12 December 2021, page 5047, XP093077128, DOI: 10.3390/rs13245047. .

### SUMMARY OF THE INVENTION

Aspects and advantages of the technology will be set forth in part in the following description, or may be obvious from the description, or may be learned through practice of the technology.

In one aspect, the present subject matter is directed to the control of an agricultural machine. The agricultural machine is a frame configured to be coupled to a tool such that the tool performs an agricultural operation on a field as the agricultural machine travels across the field. Furthermore, the agricultural machine a transceiver-based sensor configured to emit an output signal directed toward soil within a portion of the field and receive an echo signal indicative of a backscattering of the output signal by the soil. Additionally, the agricultural machine includes a computing system according to claim 1.

In another aspect, the present subject matter is directed to a computing system according to claim 1.

In a further aspect, the present subject matter is directed to a computer-implemented method according to claim 9.

These and other features, aspects and advantages of the present technology will become better understood with reference to the following description and appended claims. The accompanying drawings, which are incorporated in and constitute a part of this specification, illustrate embodiments of the technology and, together with the description, serve to explain the principles of the technology.

### BRIEF DESCRIPTION OF THE DRAWINGS

A full and enabling disclosure of the present technology, including the best mode thereof, directed to one of ordinary skill in the art, is set forth in the specification, which makes reference to the appended figures, in which:
FIG. 1 illustrates a top view of one embodiment of an agricultural machine in accordance with aspects of the present subject matter;
FIG. 2 illustrates a partial, perspective view of the agricultural machine shown in FIG. 1;
FIG. 3 illustrates a schematic view of one embodiment of a computing system in accordance with aspects of the present subject matter;
FIG. 4 illustrates a schematic view of one embodiment of a computing system in accordance with aspects of the present subject matter;
FIG. 5 illustrates a flow diagram of one embodiment of a method for determining soil moisture content in accordance with aspects of the present subject matter; and
FIG. 6 illustrates a flow diagram of one embodiment of a method for training a machine-learned model for use in determining soil moisture content in accordance with aspects of the present subject matter.

Repeat use of reference characters in the present specification and drawings is intended to represent the same or analogous features or elements of the present technology.

### DETAILED DESCRIPTION OF THE DRAWINGS

Reference now will be made in detail to embodiments of the invention, one or more examples of which are illustrated in the drawings. Each example is provided by way of explanation of the invention, not limitation of the invention. In fact, it will be apparent to those skilled in the art that various modifications and variations can be made in the present invention without departing from the scope of the invention. For instance, features illustrated or described as part of one embodiment can be used with another embodiment to yield a still further embodiment. Thus, it is intended that the present invention covers such modifications and variations as come within the scope of the appended claims. .

In general, the present subject matter is directed to systems and methods for determining the soil moisture content of an agricultural field. Specifically, in several embodiments, the disclosed systems and methods may include or otherwise leverage a machine-learned model (e.g., an unsupervised machine-learned model) to determine a soil moisture value for a portion of the field based at least in part on input data from a transceiver-based sensor. The data may be the raw data captured by the transceiver-based sensor or processed data. As such, the machine-learned model may be configured to receive the input data and process the received input data to determine or output a preliminary soil moisture value for such input data.

In several embodiments, a computing system of the disclosed system may receive data associated with backscattering that is captured by a transceiver-based sensor. Specifically, the transceiver-based sensor may be in operative association with an agricultural machine (e.g., a tractor or another agricultural vehicle and/or an associated agricultural implement) that is traveling across a field (e.g., to perform an agricultural operation thereon). In this respect, as the machine travels across the field, the transceiver-based sensor is configured to emit an output signal(s) (e.g., a microwave signal(s), such as a ground-penetrating radar (GPR) signal(s)) directed toward soil within a portion of the field and receive an echo signal(s) indicative of the backscattering of the output signal(s) by the soil. In this respect, the computing system extracts or otherwise determine a set of features, comprising at least one or more spectral components and an inverse wavelet transformation coefficient associated with the echo signal(s) from the data (e.g., from the raw data captured by the transceiver-based sensor or data captured by the transceiver-based sensor that has been processed). Thereafter, the computing system inputs the set of extracted features into the machine-learned model and, in response, receive a preliminary soil moisture value for the set of features as the output of the machine-learned model. Thereafter, the computing system determines a final soil moisture value at least in part based on the preliminary soil moisture value associated with the set of features. For example, the computing system may modify the preliminary soil moisture value based on a confidence value associated with the preliminary soil moisture value, a correction factor(s) associated with field or weather conditions, and/or the like to determine the final soil moisture value.

Additionally, the systems and methods of the present disclosure may control the operation of the agricultural machine based on the determined final soil moisture value. For example, the ground speed of the agricultural machine, the penetration depth of one or more ground-engaging tool(s) of the agricultural machine, the force applied to the ground-engaging tool(s), and/or any other suitable operating parameters of the agricultural machine may be adjusted based on the determined final soil moisture value. Moreover, when the determined final soil moisture value exceeds a maximum threshold (e.g., indicating that there is standing water or ponding in the field), an implement of the agricultural machine may be lifted up or the agricultural machine being directed around the pond/standing water). Thus, the systems and methods of the present disclosure may enable improved real-time control that improves operation of the agricultural machine and, thus, the agricultural performance of the field.

Using a machine-learned model, the systems and methods of the present disclosure determines the soil moisture content of a field with greater accuracy. These more accurate determinations of soil moisture content enable improved and/or more precise control of the agricultural machine based on current soil moisture content of the field, thereby leading to superior agricultural outcomes for the field operation(s) being performed.

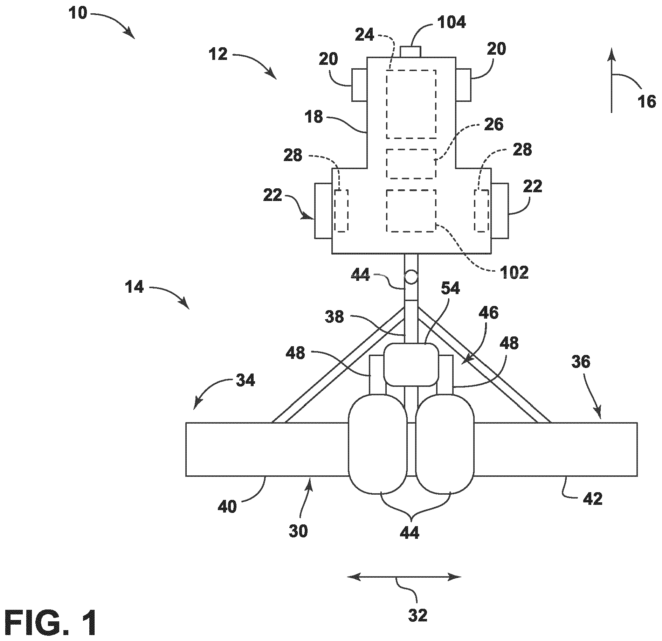

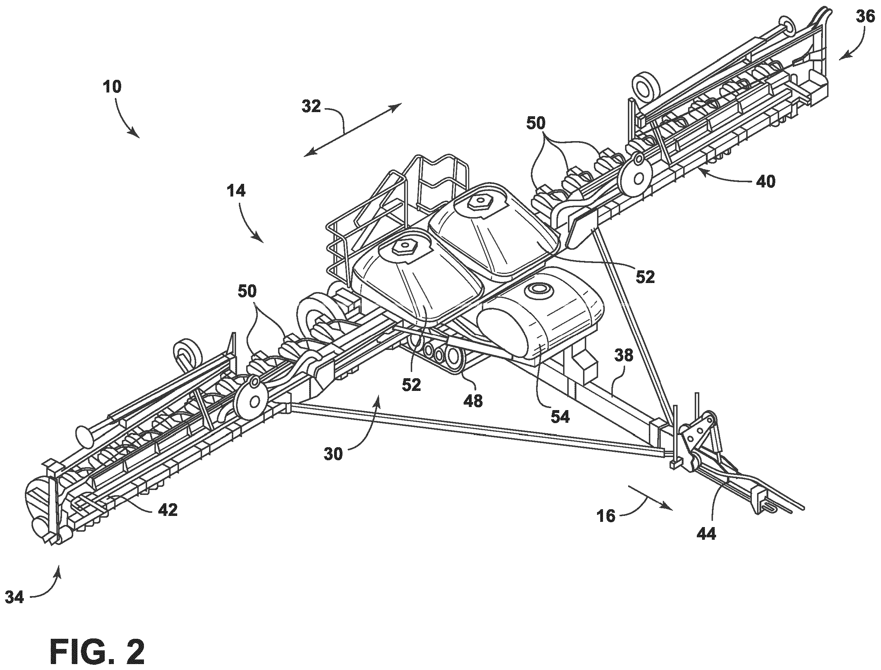

Referring now to the drawings, FIGS. 1 and 2 illustrate differing views of one embodiment of an agricultural machine 10 in accordance with aspects of the present subject matter. In the illustrated embodiment, agricultural machine 10 is configured as an agricultural vehicle 12 (e.g., an agricultural tractor) and an associated agricultural implement 14 (e.g., a seed-planting implement). In this respect, the agricultural vehicle 12 may be configured to tow the agricultural implement 14 across a field in a direction of travel (e.g., as indicated by arrow 16 in FIG. 1). However, in alternative embodiments, the agricultural machine 10 may correspond to any other suitable machine, such as any other suitable vehicle/implement combination, only an agricultural vehicle (e.g., an agricultural harvester, a self-propelled sprayer, etc.), or only an agricultural implement (e.g., a tillage implement). Additionally, in such embodiments, the agricultural machine 10 may be an unmanned aerial vehicle (UAV) suitable for use in an agricultural field.

As shown in FIG. 1, the agricultural vehicle 12 may include a frame or chassis 18 configured to support or couple to a plurality of components. For example, a pair of steerable front wheels 20 and a pair of driven rear wheels 22 may be coupled to the frame 18. The wheels 20, 22 may be configured to support the agricultural vehicle 12 relative to the ground and move the agricultural vehicle 12 in the direction of travel 16 across the field. However, in alternative embodiments, the front wheels 20 may be driven in addition to or in lieu of the rear wheels 22. Additionally, in further embodiments, the agricultural vehicle 12 may include track assemblies (not shown) in place of the front and/or rear wheels 22, 22.

Furthermore, the agricultural vehicle 12 may include one or more devices for adjusting the speed at which the agricultural vehicle 12 moves across the field in the direction of travel 16. Specifically, in several embodiments, the agricultural vehicle 12 may include an engine 24 and a transmission 26 mounted on the frame 18. In general, the engine 24 may be configured to generate power by combusting or otherwise burning a mixture of air and fuel. The transmission 26 may, in turn, be operably coupled to the engine 24 and may provide variably adjusted gear ratios for transferring the power generated by the engine power to the driven wheels 22. For example, increasing the power output by the engine 24 (e.g., by increasing the fuel flow to the engine 24) and/or shifting the transmission 26 into a higher gear may increase the speed at which the agricultural vehicle 12 moves across the field. Conversely, decreasing the power output by the engine 24 (e.g., by decreasing the fuel flow to the engine 24) and/or shifting the transmission 26 into a lower gear may decrease the speed at which the agricultural vehicle 12 moves across the field.

Additionally, the agricultural vehicle 12 may include one or more braking actuators 28 that, when activated, reduce the speed at which the agricultural vehicle 12 moves across the field, such as by converting energy associated with the movement of the agricultural vehicle 12 into heat. For example, in one embodiment, the braking actuator(s) 28 may correspond to a suitable hydraulic cylinder(s) configured to push a stationary frictional element(s) (not shown), such as a brake shoe(s) or a brake caliper(s), against a rotating element(s) (not shown), such as a brake drum(s) or a brake disc(s). However, the braking actuator(s) 28 may correspond to any other suitable hydraulic, pneumatic, mechanical, and/or electrical component(s) configured to convert the rotation of the rotating element(s) into heat. Furthermore, although FIG. 1 illustrates one braking actuator 28 provided in operative association with each of the driven wheels 22, the agricultural vehicle 12 may include any other suitable number of braking actuators 28. For example, in one embodiment, the agricultural vehicle 12 may include one braking actuator 28 provided in operative association with each of the steerable wheels 20 in addition to or in lieu of the driven wheels 22.

Moreover, a location sensor 102 may be provided in operative association with the agricultural vehicle 12 and/or the agricultural implement 14. In this regard, the location sensor 102 may be configured to detect a parameter associated with a geographical or physical location of the agricultural vehicle 12 and/or the agricultural implement 14 within the field. For instance, in one embodiment, the location sensor 102 may correspond to a GNSS-based receiver configured to detect the GNSS coordinates of the agricultural vehicle 12. However, in alternative embodiments, the location sensor 102 may be configured as any suitable location sensing device for detecting the location of the agricultural vehicle 12 and/or the agricultural implement 14.

In addition, the agricultural machine 10 may include one or more transceiver-based sensors 104. In general, the transceiver-based sensor(s) 104 is configured to emit one or more output signals directed toward the soil within a portion of the field across which the agricultural machine 10 is traveling. In one embodiment, the transceiver-based sensor may be a microwave signal-based sensor such that the output signal(s) may correspond to microwave signal(s) (e.g., a ground-penetrating radar (GPR) sensor). A portion of the output signal(s) is, in turn, backscattered or otherwise reflected by the soil as an echo signal(s). In this respect, the transceiver-based sensor(s) 104 receive the echo signal(s), which are indicative of a backscattering of the output signal(s) by the soil. As will be described below, one or more characteristics of the received echo signal(s) may be indicative of the soil moisture content of the portion of the field.

In the illustrated embodiment, a transceiver-based sensor 102 is mounted on the front end of the agricultural vehicle 12. In such an embodiment, the transceiver-based sensor 104 is configured to emit one or more output signals directed toward the soil within a portion forward of the vehicle 12 as the vehicle 12 travels across the field. However, in alternative embodiments, the transceiver-based sensor 102 may be mounted at any other suitable location, such as at another location on the agricultural vehicle 12 or on the agricultural implement 14.

Moreover, in the illustrated embodiment, the agricultural machine 10 includes a single transceiver-based sensor 104. However, in alternative embodiments, any other suitable number of transceiver-based sensors 104 may be supported on the agricultural machine 10, such as two or more transceiver-based sensors.

Referring to FIGS. 1 and 2, the implement 14 may include a frame 30 configured to support and/or couple to one or more components of the agricultural implement 14. Specifically, in several embodiments, the frame 30 may extend along a lateral direction 32 between a first side 34 of the agricultural implement 14 and a second side 36 of the agricultural implement 14. As shown, the frame 30 may include a center section 38 and a pair of wings sections 40, 42. In one embodiment, the wings sections 40, 42 may be pivotably coupled to the center section 38 to permit the wing sections 40, 42 to fold forward to reduce the lateral width of the agricultural implement 14, such as during storage or transportation of the agricultural implement 14 on a road. Furthermore, a tow bar 44 may be coupled to the center section 38 to allow the agricultural implement 14 to be towed by the agricultural vehicle 12. Moreover, a track assembly 46 having a plurality of tracks 48 may be coupled to the center section 38 to support at least a portion of the frame 30 relative to the ground. However, in alternative embodiments, the frame 30 may be supported relative to the ground by wheels (not shown) or any other suitable device.

Additionally, the wing sections 40, 42 may be configured to support a plurality of seed planting units or row units 50. In general, each row unit 50 may be configured to deposit seeds at a desired depth beneath the soil surface and at a desired seed spacing as the agricultural implement 14 is being towed by the agricultural vehicle 12, thereby establishing rows of planted seeds. In some embodiments, the bulk of the seeds to be planted may be stored in one or more hoppers or seed tanks 52 mounted on or otherwise supported by the frame 30. Thus, as seeds are planted by the row units 50, a pneumatic distribution system (not shown) may distribute additional seeds from the seed tanks 52 to the individual row units 50. Additionally, one or more fluid tanks 54 mounted on or otherwise supported by the frame 30 may store agricultural fluids, such as insecticides, herbicides, fungicides, fertilizers, and/or the like, which may be sprayed onto the seeds during planting.

For purposes of illustration, only a portion of the row units 50 of the agricultural implement 14 have been shown in FIG. 2. In general, the agricultural implement 14 may include any number of row units 50, such as six, eight, twelve, sixteen, twenty-four, thirty-two, or thirty-six row units 50. In addition, the lateral spacing between row units 50 may be selected based on the type of crop being planted. For example, the row units 50 may be spaced approximately 76.2 cm (30 inches) from one another for planting corn, and approximately 38.1 cm (15 inches) from one another for planting soybeans.

It should be appreciated that the configuration of the agricultural machine 10 described above and shown in FIGS. 1 and 2 is provided only to place the present subject matter in an exemplary field of use. Thus, it should be appreciated that the present subject matter may be readily adaptable to any manner of agricultural machine configuration.

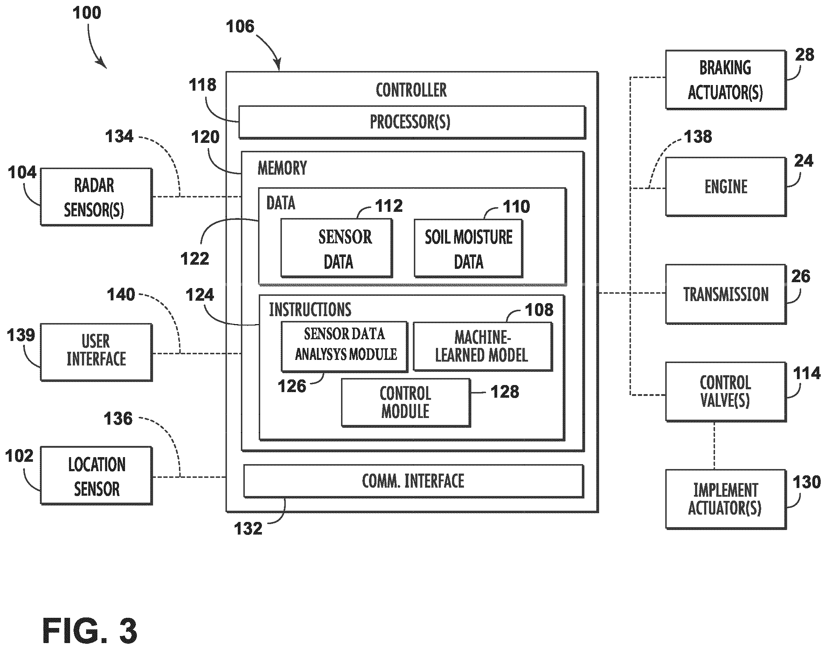

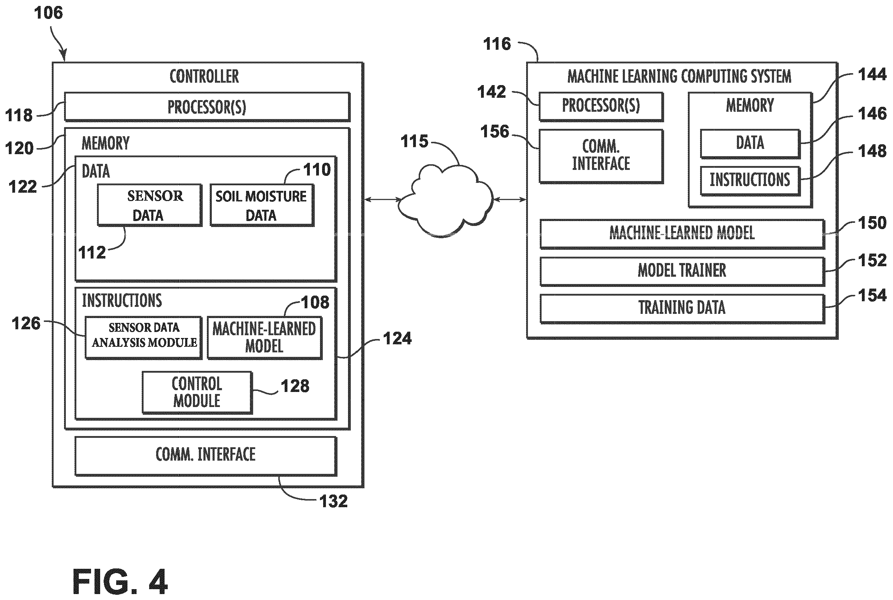

Referring now to FIGS. 3 and 4, schematic views of embodiments of a computing system 100 are illustrated in accordance with aspects of the present subject matter. In general, the system 100 will be described herein with reference to the agricultural machine 10 described above with reference to FIGS. 1 and 2. However, it should be appreciated that the disclosed system 100 may generally be utilized with agricultural machines having any suitable other suitable machine configuration.

In several embodiments, the system 100 may include a controller 106 and various other components configured to be communicatively coupled to and/or controlled by the controller 106, such as one or more transceiver-based sensors 104 and/or various components of the agricultural machine 10. In some embodiments, the controller 106 is physically coupled to or otherwise installed on the agricultural machine 10. In other embodiments, the controller 106 is not physically coupled to the agricultural machine 10 (e.g., the controller 106 may be remotely located from the agricultural machine 10) and instead may communicate with the agricultural machine 10 over a wireless network.

As will be described below, the controller 106 may be configured to leverage a machine-learned model 108 to determine the soil moisture content of the field across which the agricultural machine 10 is traveling based at least in part on data associated with the soil within the in the field that is captured by one or more transceiver-based sensor(s) 104. In particular, FIG. 3 illustrates a computing environment in which the controller 106 can operate to determine soil moisture data 110 for one or more portions of the field based on radar data 112 newly received from the one or more transceiver-based sensors 104 and, further, to control one or more components of agricultural machine 10 (e.g., the engine 24, the transmission 26, the braking actuator(s) 28, a control valve(s) 114, etc.) based on the soil moisture data 110. That is, FIG. 3 illustrates a computing environment in which the controller 106 is actively used in conjunction with an agricultural machine (e.g., during operation of the agricultural machine 10 within a field). As will be discussed below, FIG. 4 depicts a computing environment in which the controller 106 can communicate over a network 115 with a machine learning computing system 116 to train and/or receive a machine-learned model 108. Thus, FIG. 4 illustrates operation of the controller 106 to train a machine-learned model 108 and/or to receive a trained machine-learned model 108 from a machine learning computing system 116 (e.g., FIG. 4 shows the "training stage"), while FIG. 3 illustrates operation of the controller 106 to use the machine-learned model 108 to determine the soil moisture content of one or more portions of the field based on obtained radar data associated with the soil in such portion(s) of the field (e.g., FIG. 3 shows the "inference stage").

Referring first to FIG. 3, in general, the controller 106 may correspond to any suitable processor-based device(s), such as a computing device or any combination of computing devices. Thus, as shown in FIG. 3, the controller 106 may generally include one or more processor(s) 118 and associated memory devices 120 configured to perform a variety of computer-implemented functions (e.g., performing the methods, steps, algorithms, calculations and the like disclosed herein). As used herein, the term "processor" refers not only to integrated circuits referred to in the art as being included in a computer, but also refers to a controller, a microcontroller, a microcomputer, a programmable logic controller (PLC), an application specific integrated circuit, and other programmable circuits. Additionally, the memory 120 may generally comprise memory element(s) including, but not limited to, computer readable medium (e.g., random access memory (RAM)), computer readable non-volatile medium (e.g., a flash memory), a floppy disk, a compact disc-read only memory (CD-ROM), a magneto-optical disk (MOD), a digital versatile disc (DVD) and/or other suitable memory elements. Such memory 120 may generally be configured to store information accessible to the processor(s) 118, including data 122 that can be retrieved, manipulated, created and/or stored by the processor(s) 118 and instructions 124 that can be executed by the processor(s) 118.

In several embodiments, the data 122 may be stored in one or more databases. For example, the memory 120 may include a transceiver-based sensor database 112 for storing radar data received from the transceiver-based sensor(s) 104 (e.g., the raw data captured by the transceiver-based sensor(s) 104 or processed data from the transceiver-based sensor(s) 104). As described above, the transceiver-based sensor(s) 104 may be configured to continuously or periodically emit output signals directed toward soil within the field and receive echo signals indicative of the backscattering of the output signals by the soil as an agricultural operation (e.g., a seed-planting operation) is being performed within the field. In this regard, the transceiver-based sensor data (e.g., radar data) transmitted to the controller 106 from the transceiver-based sensor(s) 104, which is associated with the echo signals, may be stored within the transceiver-based sensor database 112 for subsequent processing and/or analysis. As used herein, the term "transceiver-based sensor data" may include any suitable type of microwave or radio wave-based data received from the transceiver-based sensor(s) 104 that allows for the moisture content of the soil to be determined.

Additionally, as shown in FIG. 3, the memory 120 may include a soil moisture database 110 for storing information related to the soil moisture content of one or more portions of the field. For example, as indicated above, based on the transceiver-based sensor data received from the transceiver-based sensor(s) 104, the controller 106 is configured to determine soil moisture content of the field based on one or more features associated with the echo signals, including spectral components of the echo signal and an inverse wavelet transformation coefficient of the echo signal, and optionally including the size, shape or frequency shift of the echo signal associated with the transceiver-based sensor data 112. The soil moisture determinations for one or more portions of the field made by the controller 106 may then be stored within the soil moisture database 110 for subsequent processing and/or analysis.

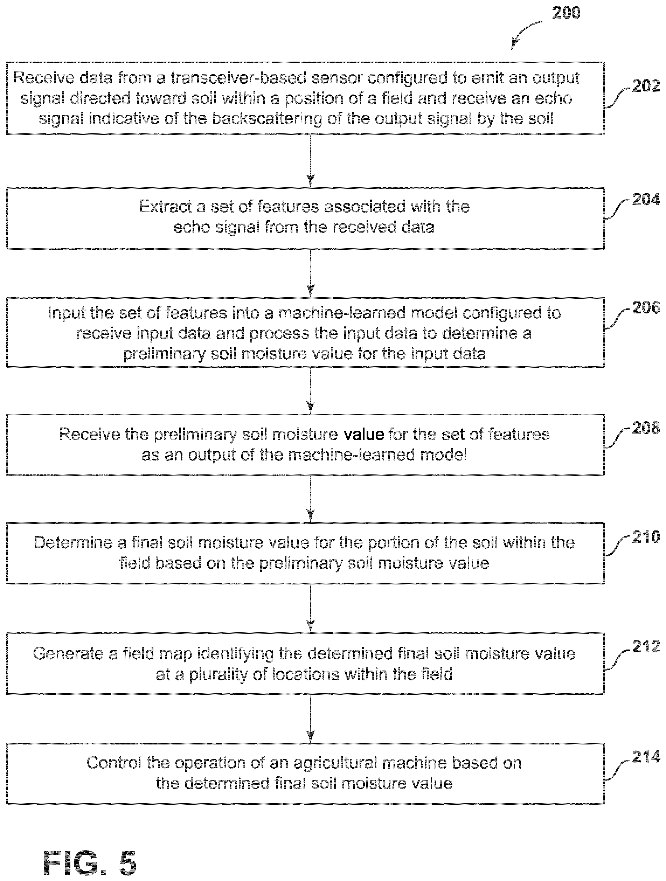

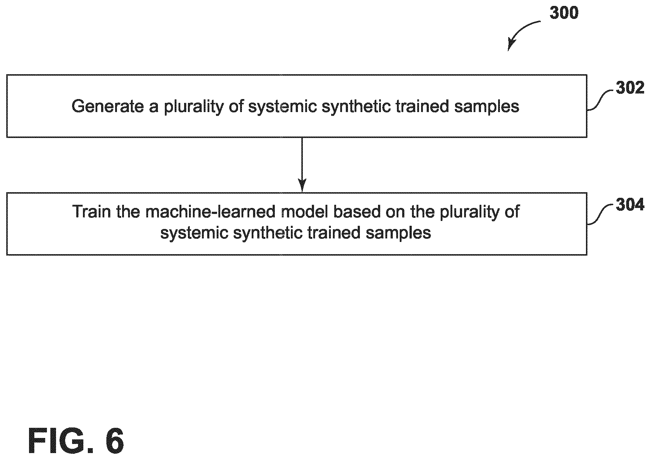

Referring still to FIG. 3, in several embodiments, the instructions 124 stored within the memory 120 of the controller 106 may be executed by the processor(s) 118 to implement a transceiver-based sensor data analysis module 126. In general, the transceiver-based sensor data analysis module 126 may be configured to analyze the transceiver-based sensor data 112 to determine the soil moisture data 110. Specifically, as will be discussed further below, the transceiver-based sensor data analysis module 126 can cooperatively operate with or otherwise leverage a machine-learned model 108 to analyze the transceiver-based sensor data 112 to determine the soil moisture data 110. For example, the transceiver-based sensor data analysis module 126 can perform some or all of method 200 illustrated in FIG. 5 and/or method 300 illustrated in FIG. 6. The controller 106 (e.g., the transceiver-based sensor data analysis module 126) may be configured to perform the above-referenced analysis for multiple portions of the field. In such instances, each portion can be analyzed individually or multiple portions can be analyzed in a batch.

Moreover, as shown in FIG. 3, the instructions 124 stored within the memory 120 of the controller 106 are executed by the processor(s) 118 to implement a machine-learned model 108. Specifically, in one embodiment, the machine-learned model 108 may be an unsupervised machine-learned model. For example, in such an embodiment, the machine-learned model 108 may be any suitable statistical regression method, including singular value decomposition (SVD), principal component analysis (PCA), hierarchical cluster analysis, K-means clustering, and/or the like. The machine-learned model 108 is configured to receive one or more sets of features extracted from radar data associated with the soil within the field. In one embodiment, the features may be extracted directly from the raw data captured by the transceiver-based sensor(s) 104. Alternatively, the raw data captured by the transceiver-based sensor(s) 104 may be processed before the features are extracted. Moreover, the machine-learned model 108 is configured to process the received set(s) of features to compute or output a preliminary soil moisture value of the radar data based on the received set(s) of features. For example, features from the transceiver-based sensor data 112 used to determine the soil moisture content may include the size or shape of the echo signals associated with the radar data. According to the claims, the features include at least one or more spectral components of the echo signal and an inverse wavelet transformation coefficient of the echo signal. In alternative embodiments, any other suitable machine learned model may be used, including supervised machine-learned models (e.g., support vector machine, K-nearest neighbors, logistic regressions, etc.).

Referring still to FIG. 3, the instructions 124 stored within the memory 120 of the controller 106 may also be executed by the processor(s) 118 to implement a control module 128. In general, the control module 128 may be configured to adjust the operation of the agricultural machine 10 by controlling one or more components of the machine 10 (e.g., of the vehicle 12 and/or the implement 14). Specifically, the control module 128 may be configured to initiate adjustments to the operation of the agricultural machine 10 based on the determined soil moisture content of the field.

In several embodiments, the control module 128 may be configured to adjust the operational or ground speed of the agricultural machine 10 based on the determined soil moisture content. In such embodiments, as shown in FIG. 3, the controller 106 may be communicatively coupled to the engine 24 and/or the transmission 26 of the vehicle 12. In this regard, the controller 106 may be configured to adjust the operation of the engine 24 and/or the transmission 26 in a manner that increases or decreases the ground speed of the agricultural machine 10, such as by transmitting suitable control signals for controlling an engine or speed governor (not shown) associated with the engine 24, transmitting suitable control signals for controlling the engagement/disengagement of one or more clutches (not shown) provided in operative association with the transmission 26, and/or transmitting suitable control signals for controlling the braking actuator(s) 28.

In addition to the adjusting the ground speed of the agricultural machine 10 (or as an alternative thereto), the control module 128 may also be configured to adjust one or more operating parameters associated with the ground-engaging tools of the agricultural machine 10 (e.g., of the implement 14). For instance, as shown in FIG. 3, the controller 106 may be communicatively coupled to one or more control valves 114 configured to regulate the supply of fluid (e.g., hydraulic fluid or air) to one or more corresponding actuators 130 (e.g., fluid-driven cylinder(s)) of the machine 10. In such an embodiment, by regulating the supply of fluid to the actuator(s) 130, the controller 106 may automatically adjust the penetration depth of and/or the force applied to the ground-engaging tools (e.g., the opener disk(s), the gauge wheel(s), closing disk(s)/wheel(s), press wheel(s), etc.) of the machine 10 (or the force applied to the row units 50).

Furthermore, in some embodiment, the control module 128 may also be configured to adjust lift up the implement 14 and/or adjust the direction of travel 16 of the agricultural machine 10 based on the determined based on the soil. For example, by regulating the supply of fluid to the actuator(s) 130, the controller 106 may automatically adjust the raise or lower the implement 14 relative to the field. Additionally, the controller 106 may be communicatively coupled to a steering actuator (not shown) of the agricultural vehicle 12. In this regard, the controller 106 may be configured to adjust the operation of the steering actuator in a manner that changes the direction of travel 16.

Moreover, as shown in FIG. 3, the controller 106 may also include a communications interface 132 to allow the controller 106 to communicate with any of the various other system components described herein. For instance, one or more communicative links or interfaces 134 (e.g., one or more data buses) may be provided between the communications interface 132 and the transceiver-based sensor(s) 104 to allow radar data transmitted from the transceiver-based sensor(s) 104 to be received by the controller 106. Similarly, one or more communicative links or interfaces 136 (e.g., one or more data buses) may be provided between the communications interface 132 and the location sensor 102 to allow location data (e.g., coordinates) transmitted from the location sensor 102 to be received by the controller 106. Moreover, one or more communicative links or interfaces 138 (e.g., one or more data buses) may be provided between the communications interface 132 and the engine 24, the transmission 26, the braking actuator(s) 28, the control valves 114, and/or the like to allow the controller 106 to control the operation of such system components.

Furthermore, in one embodiment, the computing system 100 may also include a user interface 139. More specifically, the user interface 139 may be configured to provide feedback (e.g., feedback associated with the soil moisture content of the field) to the operator of the agricultural machine 10. As such, the user interface 139 may include one or more feedback devices (not shown), such as display screens, speakers, warning lights, and/or the like, which are configured to provide feedback from the controller 106 to the operator. In addition, the user interface 139 may be configured to receive inputs from the operator. In this regard, the user interface 139 may include one or more input devices (not shown), such as touchscreens, keypads, touchpads, knobs, buttons, sliders, switches, mice, microphones, and/or the like, which are configured to receive inputs from the operator. Furthermore, one or more communicative links or interfaces 140 (e.g., one or more data buses) may be provided between the communications interface 132 of the controller 106 and the user interface 139 to allow feedback to be transmitted from the controller 106 to the user interface 139 and/or the inputs to be transmitted from the user interface 139 to the controller 106.

Referring now to FIG. 4, according to an aspect of the present disclosure, the controller 106 can store or include one or more machine-learned models 108. Specifically, in several embodiments, the machine-learned model 108 is configured to receive input data (e.g., features extracted from the radar data captured by the transceiver-based sensor(s) 104) and process the input data to determine a preliminary soil moisture value for the input data.

In some embodiments, the machine-learned model 108 may be an unsupervised machine-learned model, such as a singular value decomposition (SVD) model, a principal component analysis (PCA) model, a hierarchical cluster analysis model, a K-means clustering model, and/or the like.

In other embodiments, the machine-learned model 108 may include a supervised regression model (e.g., logistic regression classifier), a support vector machine, one or more decision-tree based models (e.g., random forest models), a Bayes classifier, a K-nearest neighbor classifier, a texton-based classifier, and/or other types of models including both linear models and non-linear models.

As an alternative, a neural network may also be used. Example neural networks include convolutional neural networks, feed-forward neural networks, recurrent neural networks (e.g., long short-term memory recurrent neural networks), or other forms of neural networks. Neural networks may include multiple connected layers of neurons, and networks with one or more hidden layers may be referred to as "deep" neural networks. Typically, at least some of the neurons in a neural network may include non-linear activation functions.

In one embodiment, the machine-learned model 108 may be configured to output a plurality of preliminary soil moisture values for the sets of features extracted from the transceiver-based sensor data 112, with each preliminary soil moisture value being attached to the set of features extracted from radar data associated with a particular portion of the field. As will be described below, the preliminary soil moisture value may be adjusted to determine a final soil moisture value that is used, e.g., to control the operation of the agricultural machine 10. Alternatively, the preliminary soil moisture value may be used as the final soil moisture value.

In some embodiments, the machine-learned model 108 may further provide, for each preliminary soil moisture value, a numerical value descriptive of a degree to which it is believed that the regression of the input data should be the corresponding preliminary soil moisture value. In some instances, the numerical values provided by the machine-learned model may be referred to as "confidence scores" that are indicative of a respective confidence associated with regression of the input into the respective preliminary soil moisture value.

In some embodiments, the controller 106 may receive the one or more machine-learned models 108 from the machine learning computing system 116 over network 115 and may store the one or more machine-learned models 108 in the memory 120. The controller 106 may then use or otherwise run the one or more machine-learned models 108 (e.g., via the processor(s) 118).

The machine learning computing system 116 may include one or more processors 142 and a memory 144. The one or more processors 142 may be any suitable processing device such as those described with reference to the processor(s) 118. The memory 144 may include any suitable storage device(s) such as those described with reference to memory 120.

The memory 144 may store information that can be accessed by the one or more processors 142. For instance, the memory 144 (e.g., one or more non-transitory computer-readable storage mediums, memory devices) may store data 146 that can be obtained, received, accessed, written, manipulated, created, and/or stored. In some embodiments, the machine learning computing system 116 may obtain data from one or more memory device(s) that are remote from the system 116. Furthermore, the memory 144 may also store computer-readable instructions 148 that can be executed by the processor(s) 142. The instructions 148 may, in turn, be software written in any suitable programming language or may be implemented in hardware. Additionally, or alternatively, the instructions 148 can be executed in logically and/or virtually separate threads on processor(s) 142. For example, the memory 144 may store instructions 148 that when executed by the processor(s) 142 cause the processor(s) 142 to perform any of the operations and/or functions described herein.

In some embodiments, the machine learning computing system 116 may include one or more server computing devices. When the machine learning computing system 116 includes multiple server computing devices, such server computing device(s) may operate according to various computing architectures, including, for example, sequential computing architectures, parallel computing architectures, or some combination thereof.

In addition to or as an alternative to the model(s) 108 at the controller 106, the machine learning computing system 116 can include one or more machine-learned models 150. For example, the model(s) 150 may be the same as described above with reference to the model(s) 108.

In some embodiments, the machine learning computing system 116 may communicate with the controller 106 according to a client-server relationship. For example, the machine learning computing system 116 may implement the machine-learned models 150 to provide a web service to the controller 106. For example, the web service can provide radar data analysis for soil moisture determination as a service. Thus, machine-learned models 108 can be located and used at the controller 106 and/or machine-learned models 150 can be located and used at the machine learning computing system 116.

In some embodiments, the machine learning computing system 116 and/or the controller 106 may train the machine-learned models 108 and/or 150 through use of a model trainer 152. The model trainer 152 may train the machine-learned models 108 and/or 150 using one or more training or learning algorithms. One example training technique is backwards propagation of errors ("backpropagation"). Other training techniques may be used. In several embodiments, the model trainer 152 may train the machine-learned models 108 and/or 150 using a plurality of systematic synthetic trained samples.

In some embodiments, the model trainer 152 may perform supervised training techniques using a set of labeled training data 154. For example, the labeled training data 154 may include sets of features, with each set of features being labeled (e.g., manually by an expert and/or manually by a user of the models) with a "correct" or ground-truth label. Thus, each training example may include a set of features and a corresponding ground-truth classification for the set of features. The labels used for the training data 154 may match any of the example labelling schemes described herein, including continuous labels (e.g., various soil moisture values) or other labelling schemes.

In other embodiments, the model trainer 152 may perform unsupervised training techniques using a set of unlabeled training data 154. The model trainer 152 may perform a number of generalization techniques to improve the generalization capability of the models being trained. Generalization techniques include weight decays, dropouts, or other techniques. The model trainer 152 may be implemented in hardware, software, firmware, or combinations thereof.

Thus, in some embodiments, the model(s) may be trained at a centralized computing system (e.g., at "the factory") and then distributed to (e.g., transferred to for storage by) specific controllers. Additionally, or alternatively, the models can be trained (or re-trained) based on additional training data generated by the user. This process may be referred to as "personalization" of the model(s) and may allow the operator to further train the models to provide improved (e.g., more accurate) predictions for unique field conditions experienced by the operator.

The network(s) 115 may be any type of network or combination of networks that allows for communication between devices. In some embodiments, the network(s) 115 may include one or more of a local area network, wide area network, the Internet, secure network, cellular network, mesh network, peer-to-peer communication link and/or some combination thereof and can include any number of wired or wireless links. Communication over the network(s) 115 may be accomplished, for instance, via a communications interface using any type of protocol, protection scheme, encoding, format, packaging, etc. Additionally, the machine learning computing system 116 may also include a communications interface to communicate with any of the various other system components described herein.

FIGS. 3 and 4 illustrate example computing systems that may be used to implement the present disclosure. Other computing systems may be used as well. For example, in some embodiments, the controller 106 may include the model trainer 152 and the training dataset 154. In such embodiments, the machine-learned model(s) 108 may be both trained and used locally at the controller 106. As another example, in some embodiments, the controller 106 may not connected to other computing systems.

Referring now to FIG. 5, a flow diagram of one embodiment of a method 200 for determining soil moisture content for a field based on radar data is illustrated in accordance with aspects of the present subject matter. In general, the method 200 will be described herein with reference to the agricultural machine 10 shown in FIGS. 1 and 2, as well as the various system components shown in FIGS. 3 and/or 4. However, it should be appreciated that the disclosed method 200 may be implemented with agricultural machines having any other suitable machine configuration and/or within systems having any other suitable system configuration.

As shown in FIG. 5, at (202), the method 200 includes receiving data from a transceiver-based sensor 104. As described above, the agricultural machine 10 may include one or more transceiver-based sensors 104 configured to emit an output signal(s) directed toward soil within a portion of a field. The soil, in turn, backscatters or otherwise reflects a portion of the output signal(s) as an echo signal(s), with the soil moisture content of the portion of the field affecting the characteristics of the echo signal(s). As such, the transceiver-based sensor(s) 104 is configured to receive the backscattered echo signal(s). Furthermore, the controller 106 may be communicatively coupled to the transceiver-based sensor(s) 104 via the communicative link 134. In this respect, as the agricultural machine 10 travels across a field (e.g., to perform an agricultural operation, such as a seed-planting operation, thereon), the controller 106 is configured to receive data from the transceiver-based sensor(s) 104 that is indicative of the received echo signal(s). Such received data may be the raw data captured by the transceiver-based sensor(s) 102 or data captured by the transceiver-based sensor(s) 102 that has been processed. As will be described below, the received transceiver-based sensor data is generally used to determine the soil moisture content of the field.

Additionally, at (204), the method 200 includes extracting one or more sets of features associated with the echo signal(s) from the received radar data. In general, each set of features extracted from the radar data may be associated with the echo signal(s) received by the transceiver-based sensor(s) 104. Specifically, in several embodiments, the transceiver-based sensor data analysis module 126 of the controller 106 may be configured to analyze the received transceiver-based sensor data to extract one or more sets of features associated with. In some embodiments, the features are extracted directly from the raw data captured by the transceiver-based sensor(s) 104. Alternatively, the features may be extracted processed data. Such set(s) of features may, in turn, be affected by varying levels of moisture content within the soil and, thus, be used to determine the soil moisture content of the field. For example, the features may include the size (e.g., the amplitude) of the echo signal(s), the shape of the echo signal(s) or the frequency shift of the echo signal(s). According to the claims, the feature comprises at least one or more spectral components of the echo signal(s) and the inverse wavelet transformation coefficient of the echo signal(s). As such, the radar data analysis module 126 may use a suitable algorithm(s) to extract the features from the radar data.

As shown in FIG. 5, at (206), the method 200 includes inputting the set of features into a machine-learned model. As described above, the machine-learned model 108 is configured to receive input data and process the input data to determine a preliminary soil moisture value for the input data. As such, the transceiver-based sensor data analysis module 126 of the controller 106 may be configured to input the sets of extracted features into the machine-learned model 108. In some embodiments, the inputted sets of features may correspond to or otherwise have been extracted from an entirety of the radar data, such that all of the transceiver-based sensor data is analyzed. In other embodiments, the inputted sets of features may correspond to or otherwise have been extracted only a portion or subset of the transceiver-based sensor data. Using only a subset of the transceiver-based sensor data may enable reductions in processing time and requirements.

Additionally, at (208), the method 200 includes receiving a preliminary soil moisture value for a portion of the field as an output of the machine-learned model. For example, as indicated above, the transceiver-based sensor data analysis module 126 of the controller 106 may be configured to receive a respective preliminary soil moisture value for the set(s) of extracted features as an output of the machine-learned model 108. As described above, in some embodiments, each preliminary soil moisture value may be a continuous value or number for the soil moisture content of a given of the field. As will be described below, received preliminary soil moisture value may be adjusted to determine a final soil moisture value for the given of the field. However, other labeling schemes can be used in addition or alternatively to these example schemes.

Moreover, at (208), the method 200 includes obtaining a respective confidence score associated with each preliminary soil moisture value. For example, the transceiver-based sensor data analysis module 126 of the controller 106 may be configured to receive the confidence scores as a further output from the machine-learned model 108 respectively alongside the preliminary soil moisture value output for the sets of extracted features. Specifically, in some embodiments, the machine-learned model can output a confidence scores for each set of features extracted from the received transceiver-based sensor data. As example, the confidence values may be a range of values between zero (indicating no confidence in the preliminary soil moisture value) and one (indicating complete confidence in the preliminary soil moisture value).

As shown in FIG. 5, at (210), the method 200 includes determining a final soil moisture value for the portion of the soil within the field based on the preliminary soil moisture value for the set of extracted features. For example, the transceiver-based sensor data analysis module 126 of the controller 106 may be configured to determine final soil moisture value for the portion of the soil within the field based on the received preliminary soil moisture value(s) for the set(s) of features extracted from the transceiver-based sensor data 112. In several embodiments, the transceiver-based sensor data analysis module 126 of the controller 106 may be configured to adjust or otherwise modify the received preliminary soil moisture value(s) based on one or more correction factors to determine the final soil moisture value(s). For example, such correction factor(s) may be based on the soil type of the field, the time of year, the crops growing in the field, recent weather conditions, and/or the like. Additionally, the transceiver-based sensor data analysis module 126 of the controller 106 may be configured to determine the final soil moisture value(s) based on the confidence value(s) associated with the preliminary soil moisture value(s). Thus, the final soil moisture value(s) may take into account other factors that are not part of the set(s) of features provided to the machine-learned model 108. In other embodiments, the transceiver-based sensor data analysis module 126 of the controller 106 may use the received preliminary soil moisture value(s) as the final soil moisture value(s).

In addition, after determining the final soil moisture value at (210), the method 200 may include, at (212), generating a field map identifying the determined final soil moisture value at a plurality of locations within the field. Specifically, in several embodiment, the controller 106 may be configured to correlate each determined final soil moisture value to a corresponding set of coordinates received from the location sensor 102 via the communicative link 136. Thereafter, the controller 106 may generate a field map identifying the determined final soil moisture value at a plurality of locations within the field.

Furthermore, after determining the final soil moisture value at (210), the method 200 may include, at (214), controlling the operation of an agricultural machine based on the determined final soil moisture value. Specifically, as indicated above, the control module 128 of the controller 106 may be configured to control the operation of the agricultural machine 10 (e.g., the vehicle 12 and/or the implement 14) to adjust one or more operating parameters of the agricultural machine 10 based on the final soil moisture value(s). In some embodiments, the control module 128 may be configured to initiate an adjustment of the ground speed of the agricultural vehicle 12, the penetration depth of the ground-engaging tool(s) of the implement 14, and/or the force applied to the tool(s) based on the determined final soil moisture value(s). For example, in one embodiment, the control module 128 may be configured to initiate an adjustment of the furrow depth (e.g., by controlling the control valve(s) 114) and, thus, the depth of the seeds being planted within the field based on the determined final soil moisture value(s).

Additionally, in several embodiments, when a pond or other standing water is present within the field the implement 14 may be lifted up or the agricultural machine 10 may travel around the pond/standing water. More specifically, when the determined final soil moisture value exceeds a maximum threshold, there may be standing water or a pond in the field. In such instances, the control module 128 of the controller 106 may be configured to control the operation of the agricultural machine 10 (e.g., the vehicle 12 and/or the implement 14) to lift the implement 14 up out of the water when the agricultural machine 10 travels through the standing water. Alternatively, e control module 128 of the controller 106 may be configured to control the operation of the agricultural machine 10 (e.g., the vehicle 12 and/or the implement 14) to adjust the direction of travel 16 such that the agricultural machine 10 travels around the pond/standing water.

Referring now to FIG. 6, a flow diagram of one embodiment of a method 300 for training a machine-learned model for use in determining soil moisture content of a field is illustrated in accordance with aspects of the present subject matter. In general, the method 300 will be described herein with reference to the agricultural machine 10 shown in FIGS. 1 and 2, as well as the various system components shown in FIGS. 3 and/or 4. However, it should be appreciated that the disclosed method 300 may be implemented with agricultural machines having any other suitable machine configuration and/or within systems having any other suitable system configuration. In addition, although FIG. 6 depicts steps performed in a particular order for purposes of illustration and discussion, the methods discussed herein are not limited to any particular order or arrangement. One skilled in the art, using the disclosures provided herein, will appreciate that various steps of the methods disclosed herein can be omitted, rearranged, combined, and/or adapted in various ways without deviating from the scope of the present disclosure.

As shown, at (302), the method 300 may include generating a plurality of systematic synthetic trained samples. As described above, the controller 106 leverages a machine-learned model 108 to determine the soil moisture content of a field based on transceiver-based sensor data 112. In this respect, and as will be described below, the machine learning computing system 116 may be configured to train the machine-learned model 108 to output a preliminary soil moisture value for a given set of features extracted from the transceiver-based sensor data 112 using the training data 154. In several embodiment, the machine learning computing system 116 may be configured to generate a plurality of systematic synthetic trained samples to form the training data 154. Specifically, the machine learning computing system 116 may create a simulated environment (e.g., a simulated agricultural field) from which the machine learning computing system 116 systematically creates artificial sets of features (e.g., thousands or tens of thousands of sets) that can be used to train the machine-learned model 108. Such systematic synthetic trained samples simulate possible field conditions without the need for real-world data samples, thereby allowing the machine learning computing system 116 to amass the training data much quicker than when relying on real-world data samples. That is, the systematic synthetic trained samples are generated or simulated by the machine learning computing system 116 and are not real-world data samples.

Furthermore, as shown in FIG. 6, at (304), the method may include training the machine-learned model based on the systematic synthetic trained samples. As described above, in certain embodiments, the machine learning computing system 116 may include the model trainer 152. As such, in one embodiment, the model trainer 152 may be configured to train the machine-learned model 108 (e.g., using unsupervised methods) based on the systematic synthetic trained samples generated at (302). Obtaining real world data samples of radar data for determining soil moisture content is extremely time-consuming and expensive. Moreover, it is not realistic to obtain such samples without extensive time in the field, such as after the purchase of the agricultural machine 10. As such, by using the artificially- and systematically created synthetic trained samples as opposed to real word data samples, the machine-learned model 108 can be trained quickly and at the factory and without the need for extensive field time, thereby allowing the machine-learned model 108 to make more accurate preliminary soil moisture value determinations after a shorter training period.

It is to be understood that the steps of the methods 200, 300 are performed by the computing system 100 upon loading and executing software code or instructions which are tangibly stored on a tangible computer readable medium, such as on a magnetic medium, e.g., a computer hard drive, an optical medium, e.g., an optical disc, solid-state memory, e.g., flash memory, or other storage media known in the art. Thus, any of the functionality performed by the computing system 100 described herein, such as the methods 200, 300, is implemented in software code or instructions which are tangibly stored on a tangible computer readable medium. The computing system 100 loads the software code or instructions via a direct interface with the computer readable medium or via a wired and/or wireless network. Upon loading and executing such software code or instructions by the computing system 100, the computing system 100 may perform any of the functionality of the computing system 100 described herein, including any steps of the methods 200, 300 described herein.

## Claims

1. A computing system (100), comprising one or more processors (118) and one or more non-transitory computer-readable media (120), the one or more non-transitory computer-readable media (120) collectively storing:
a machine-learned model (108) configured to receive input data and process the input data to determine a preliminary soil moisture value for the input data; and
instructions (124) that, when executed by the one or more processors (118), configure the computing system (100) to perform operations, the operations comprising:
receiving data from a transceiver-based sensor (104) configured to emit an output signal directed toward soil within a portion of a field and receive an echo signal indicative of a backscattering of the output signal by the soil;
extracting a set of features associated with the echo signal from the received data;
inputting the set of features into the machine-learned model (108);
receiving the preliminary soil moisture value for the set of features as an output of the machine-learned model (108); and
determining a final soil moisture value for the portion of the soil within the field based on the preliminary soil moisture value,
the machine-learned model (108) is configured to output a confidence score for the preliminary soil moisture value for the set of features; and
determining the final soil moisture value comprises determining the final soil moisture value for the portion of the soil within the field based on the confidence score and the preliminary soil moisture value.
**characterized in that**
extracting the set of features comprises determining one or more spectral components of the echo signal and an inverse wavelet transformation coefficient of the echo signal.

2. The computing system (100) as in claim 1, wherein the operations further comprise training the machine-learned model (108) based on a plurality of systematic synthetic trained samples.

3. The computing system (100) as in any preceding claim, wherein the machine-learned model (108) comprises an unsupervised machine-learned model.

4. The computing system (100) as in any preceding claim, wherein the operations further comprise controlling an operation of an agricultural machine (10) based on the determined final soil moisture value.

5. The computing system (100) as in any preceding claim, wherein controlling the operation of the agricultural machine (10) comprises initiating an adjustment to a ground speed of the agricultural machine (10) based on the determined final soil moisture value.

6. The computing system (100) as in any preceding claim, wherein controlling the operation of the agricultural machine (10) comprises initiating an adjustment to a penetration depth of a ground-engaging tool (50) of the agricultural machine (10) based on the determined final soil moisture value.

7. The computing system (100) as in any preceding claim, wherein controlling the operation of the agricultural machine (10) comprises initiating at least one of a change in the direction of travel of the agricultural machine (10) or lifting up an implement (14) of the agricultural machine (10).

8. The computing system (100) as in any preceding claim, wherein the operations further comprise generating a field map identifying the determined final soil moisture value at a plurality of locations within the field.

9. A computer-implemented method (200, 300), comprising:
receiving, with a computing system (100) comprising one or more computing devices (106, 116), data from a transceiver-based sensor (104) configured to emit an output signal directed toward soil within a portion of a field and receive an echo signal indicative of a backscattering of the output signal by the soil;
extracting, with the computing system (100), a set of features associated with the echo signal from the received data;
inputting, with the computing system (100), the set of features into a machine-learned model (108) configured to receive input data and process the input data to determine a preliminary soil moisture value for the input data;
receiving, with the computing system (100), the preliminary soil moisture value for the set of features as an output of the machine-learned model (108); and
determining, with the computing system (100), a final soil moisture value for the portion of the soil within the field based on the preliminary soil moisture value
the machine-learned model (108) is configured to output a confidence score for the preliminary soil moisture value for the set of features; and
determining the final soil moisture value comprises determining, with the computing system (100), the final soil moisture value for the portion of the soil within the field based on the confidence score and the preliminary soil moisture value; **characterized in that**
extracting the set of features comprises determining one or more spectral components of the echo signal, and an inverse wavelet transformation coefficient of the echo signal.

10. The computer-implemented method (200, 300) as in claim 9, further comprising training the machine-learned model (108) based on a plurality of systematic synthetic trained samples.

11. The computer-implemented method (200, 300) as in any of claims 9 or 10, wherein the machine-learned model (108) comprises an unsupervised machine-learned model.

## Patentansprüche

1. Rechensystem (100), umfassend einen oder mehrere Prozessoren (118) und ein oder mehrere nicht-transitorische computerlesbare Medien (120), wobei das eine oder die mehreren nicht-transitorischen computerlesbaren Medien (120) kollektiv speichern:
ein maschinell gelerntes Modell (108), das konfiguriert ist, um Eingabedaten zu empfangen und die Eingabedaten zu verarbeiten, um einen vorläufigen Bodenfeuchtigkeitswert für die Eingabedaten zu bestimmen; und
Anweisungen (124), die, wenn sie von dem einen oder den mehreren Prozessoren (118) ausgeführt werden, das Rechensystem (100) zur Durchführung von Operationen konfigurieren, wobei die Operationen umfassen:
Empfangen von Daten von einem transceiverbasierten Sensor (104), der konfiguriert ist, um ein Ausgabesignal abzugeben, das auf Boden innerhalb eines Abschnitts eines Feldes gerichtet ist, und ein Echosignal zu empfangen, das eine Rückstreuung des Ausgabesignals durch den Boden anzeigt;
Extrahieren eines Satzes von Merkmalen, die mit dem Echosignal assoziiert sind, aus den empfangenen Daten;
Eingeben des Satzes von Merkmalen in das maschinell gelernte Modell (108);
Empfangen des vorläufigen Bodenfeuchtigkeitswerts für den Satz von Merkmalen als eine Ausgabe des maschinell gelernten Modells (108) und
Bestimmen eines finalen Bodenfeuchtigkeitswerts für den Abschnitt des Bodens innerhalb des Felds basierend auf dem vorläufigen Bodenfeuchtigkeitswert,
das maschinell gelernte Modell (108) konfiguriert ist, um einen Konfidenzwert für den vorläufigen Bodenfeuchtigkeitswert für den Satz von Merkmalen auszugeben; und
das Bestimmen des finalen Bodenfeuchtigkeitswerts das Bestimmen des finalen Bodenfeuchtigkeitswerts für den Abschnitt des Bodens innerhalb des Felds basierend auf dem Konfidenzwert und dem vorläufigen Bodenfeuchtigkeitswert umfasst.
**dadurch gekennzeichnet, dass**
Extrahieren des Satzes von Merkmalen das Bestimmen einer oder mehrerer spektraler Komponenten des Echosignals und eines inversen Wavelet-Transformationskoeffizienten des Echosignals umfasst.

2. Rechensystem (100) nach Anspruch 1, wobei die Operationen ferner das Trainieren des maschinell gelernten Modells (108) basierend auf einer Vielzahl von systematischen synthetischen Trainingsproben umfassen.

3. Rechensystem (100) nach einem der vorstehenden Ansprüche, wobei das maschinell gelernte Modell (108) ein unüberwachtes maschinell gelerntes Modell umfasst.

4. Rechensystem (100) nach einem der vorstehenden Ansprüche, wobei die Operationen ferner das Steuern einer Operation einer Landmaschine (10) basierend auf dem bestimmten finalen Bodenfeuchtigkeitswert umfassen.

5. Rechensystem (100) nach einem der vorstehenden Ansprüche, wobei das Steuern des Betriebs der Landmaschine (10) das Einleiten einer Anpassung einer Bodengeschwindigkeit der Landmaschine (10) basierend auf dem bestimmten finalen Bodenfeuchtigkeitswert umfasst.

6. Rechensystem (100) nach einem der vorstehenden Ansprüche, wobei das Steuern des Betriebs der Landmaschine (10) das Einleiten einer Anpassung einer Eindringtiefe eines bodeneingreifenden Werkzeugs (50) der Landmaschine (10) basierend auf dem bestimmten finalen Bodenfeuchtigkeitswert umfasst.

7. Rechensystem (100) nach einem der vorstehenden Ansprüche, wobei das Steuern des Betriebs der Landmaschine (10) das Einleiten mindestens eines von einer Änderung der Fahrtrichtung der Landmaschine (10) oder dem Anheben eines Arbeitsgeräts (14) der Landmaschine (10) umfasst.

8. Rechensystem (100) nach einem der vorstehenden Ansprüche, wobei die Operationen ferner das Erzeugen einer Feldkarte umfassen, die den bestimmten finalen Bodenfeuchtigkeitswert an einer Vielzahl von Standorten innerhalb des Feldes identifiziert.

9. Computerimplementiertes Verfahren (200, 300), umfassend:
Empfangen, mit einem Rechensystem (100), das eine oder mehrere Rechenvorrichtungen (106, 116) umfasst, von Daten von einem transceiverbasierten Sensor (104), der konfiguriert ist, um ein Ausgabesignal abzugeben, das auf Boden innerhalb eines Abschnitts eines Feldes gerichtet ist, und ein Echosignal zu empfangen, das eine Rückstreuung des Ausgabesignals durch den Boden anzeigt;
Extrahieren, mit dem Rechensystem (100), eines Satzes von Merkmalen, die mit dem Echosignal assoziiert sind, aus den empfangenen Daten;
Eingeben, mit dem Rechensystem (100), des Satzes von Merkmalen in ein maschinell gelerntes Modell (108), das konfiguriert ist, um Eingabedaten zu empfangen und die Eingabedaten zu verarbeiten, um einen vorläufigen Bodenfeuchtigkeitswert für die Eingabedaten zu bestimmen;
Empfangen, mit dem Rechensystem (100), des vorläufigen Bodenfeuchtigkeitswerts für den Satz von Merkmalen als eine Ausgabe des maschinell gelernten Modells (108) und
Bestimmen, mit dem Rechensystem (100), eines finalen Bodenfeuchtigkeitswerts für den Abschnitt des Bodens innerhalb des Felds basierend auf dem vorläufigen Bodenfeuchtigkeitswert,
das maschinell gelernte Modell (108) konfiguriert ist, um einen Konfidenzwert für den vorläufigen Bodenfeuchtigkeitswert für den Satz von Merkmalen auszugeben; und
das Bestimmen des finalen Bodenfeuchtigkeitswerts das Bestimmen, mit dem Rechensystem (100), des finalen Bodenfeuchtigkeitswerts für den Abschnitt des Bodens innerhalb des Felds basierend auf dem Konfidenzwert und dem vorläufigen Bodenfeuchtigkeitswert umfasst; **dadurch gekennzeichnet, dass**
Extrahieren des Satzes von Merkmalen das Bestimmen einer oder mehrerer spektraler Komponenten des Echosignals und eines inversen Wavelet-Transformationskoeffizienten des Echosignals umfasst.

10. Computerimplementiertes Verfahren (200, 300) nach Anspruch 9, ferner umfassend das Trainieren des maschinell gelernten Modells (108) basierend auf einer Vielzahl von systematischen synthetischen Trainingsproben.

11. Computerimplementiertes Verfahren (200, 300) nach einem der Ansprüche 9 oder 10, wobei das maschinell gelernte Modell (108) ein unüberwachtes maschinell gelerntes Modell umfasst.

## Revendications

1. Système informatique (100), comprenant un ou plusieurs processeurs (118) et un ou plusieurs supports non transitoires lisibles par un ordinateur (120), le ou les supports non transitoires lisibles par un ordinateur (120) stockant collectivement :
un modèle appris par machine (108) configuré pour recevoir des données d'entrée et traiter les données d'entrée pour déterminer une valeur préliminaire d'humidité de sol pour les données d'entrée ; et
des instructions (124) qui, lorsqu'elles sont exécutées par le ou les processeurs (118), configurent le système informatique (100) pour mettre en œuvre des opérations, les opérations comprenant :
la réception de données à partir d'un capteur basé sur un émetteur-récepteur (104) configuré pour émettre un signal de sortie dirigé vers le sol situé dans une partie d'un champ et pour recevoir un signal d'écho indiquant une rétrodiffusion du signal de sortie par le sol ;
l'extraction d'un ensemble de caractéristiques associées au signal d'écho à partir des données reçues ;
l'entrée de l'ensemble de caractéristiques dans le modèle appris par machine (108) ;
la réception de la valeur préliminaire d'humidité de sol pour l'ensemble de caractéristiques en tant que sortie du modèle appris par machine (108) ; et
la détermination d'une valeur finale d'humidité de sol pour la partie du sol située dans le champ sur la base de la valeur préliminaire d'humidité de sol,
le modèle appris par machine (108) est configuré pour délivrer en sortie un score de confiance pour la valeur préliminaire d'humidité de sol pour l'ensemble de caractéristiques ; et
la détermination de la valeur finale d'humidité de sol comprend la détermination de la valeur finale d'humidité de sol pour la partie du sol située dans le champ sur la base du score de confiance et de la valeur préliminaire d'humidité de sol.
**caractérisé en ce que**
l'extraction de l'ensemble de caractéristiques comprend la détermination d'une ou plusieurs composantes spectrales du signal d'écho et d'un coefficient de transformation en ondelettes inverses du signal d'écho.

2. Système informatique (100) selon la revendication 1, dans lequel les opérations comprennent en outre l'entraînement du modèle appris par machine (108) sur la base d'une pluralité d'échantillons entraînés synthétiques systématiques.

3. Système informatique (100) selon l'une quelconque revendication précédente, dans lequel le modèle appris par machine (108) comprend un modèle appris par machine non supervisé.

4. Système informatique (100) selon l'une quelconque revendication précédente, dans lequel les opérations comprennent en outre la commande d'une opération d'une machine agricole (10) sur la base de la valeur finale d'humidité de sol déterminée.

5. Système informatique (100) selon l'une quelconque revendication précédente, dans lequel la commande de l'opération de la machine agricole (10) comprend l'initiation d'un ajustement à une vitesse au sol de la machine agricole (10) sur la base de la valeur finale d'humidité de sol déterminée.

6. Système informatique (100) selon l'une quelconque revendication précédente, dans lequel la commande de l'opération de la machine agricole (10) comprend l'initiation d'un ajustement à une profondeur de pénétration d'un outil entrant en contact avec le sol (50) de la machine agricole (10) sur la base de la valeur finale d'humidité de sol déterminée.

7. Système informatique (100) selon l'une quelconque revendication précédente, dans lequel la commande du fonctionnement de la machine agricole (10) comprend l'initiation d'au moins l'un parmi un changement dans le sens de déplacement de la machine agricole (10) ou le soulèvement d'un outil (14) de la machine agricole (10).

8. Système informatique (100) selon l'une quelconque revendication précédente, dans lequel les opérations comprennent en outre la génération d'une carte de terrain identifiant la valeur finale d'humidité de sol déterminée à une pluralité d'endroits dans le champ.

9. Procédé mis en œuvre par ordinateur (200, 300), comprenant :
la réception, avec un système informatique (100) comprenant un ou plusieurs dispositifs informatiques (106, 116), de données à partir d'un capteur basé sur un émetteur-récepteur (104) configuré pour émettre un signal de sortie dirigé vers le sol dans une partie d'un champ et recevoir un signal d'écho indiquant une rétrodiffusion du signal de sortie par le sol ;
l'extraction, avec le système informatique (100), d'un ensemble de caractéristiques associées au signal d'écho à partir des données reçues ;
l'entrée, avec le système informatique (100), de l'ensemble de caractéristiques dans un modèle appris par machine (108) configuré pour recevoir des données d'entrée et traiter les données d'entrée pour déterminer une valeur préliminaire d'humidité de sol pour les données d'entrée ;
la réception, avec le système informatique (100), de la valeur préliminaire d'humidité de sol pour l'ensemble de caractéristiques en tant que sortie du modèle appris par machine (108) ; et
la détermination, à l'aide du système informatique (100), d'une valeur finale d'humidité du sol pour la partie du sol située dans le champ sur la base de la valeur préliminaire d'humidité du sol
le modèle appris par machine (108) est configuré pour délivrer en sortie un score de confiance pour la valeur préliminaire d'humidité de sol pour l'ensemble de caractéristiques ; et
la détermination de la valeur finale d'humidité de sol comprend la détermination, avec le système informatique (100), de la valeur finale d'humidité de sol pour la partie du sol située dans le champ sur la base du score de confiance et de la valeur préliminaire d'humidité de sol ; **caractérisé en ce que**
l'extraction de l'ensemble de caractéristiques comprend la détermination d'une ou plusieurs composantes spectrales du signal d'écho et d'un coefficient de transformation en ondelettes inverses du signal d'écho.

10. Procédé mis en œuvre par ordinateur (200, 300) selon la revendication 9, comprenant en outre un entraînement du modèle appris par machine (108) sur la base d'une pluralité d'échantillons entraînés synthétiques systématiques.

11. Procédé mis en œuvre par ordinateur (200, 300) selon l'une quelconque des revendications 9 ou 10, dans lequel le modèle appris par machine (108) comprend un modèle appris par machine non supervisé.
